# FASCICULE DE BREVET EUROPEEN

(11) **EP 2 454 742 B1**
(45) Date de publication et mention de la délivrance du brevet: **23.01.2013**
(21) Numéro de dépôt: 10751978.7
(22) Date de dépôt: 16.07.2010
(51) Int. Cl.: H01F 5/00

(54) **DISPOSITIF CRÉANT UN CHAMP MAGNÉTIQUE D'ABORD VARIABLE À FRÉQUENCE ÉQUIVALENTE AU RYTHME DELTA, PUIS CONSTANT AFIN QUE DÈS LORS L'UTILISATEUR DORME COMME PLACÉ SUR LE PÔLE MAGNÉTIQUE NORD - OU SUD**
VORRICHTUNG ZUR ERZEUGUNG EINES ZUNÄCHST ENTSPRECHEND DER DELTAWELLENFREQUENZ VARIABLEN UND ANSCHLIESSEND KONSTANTEN MAGNETISCHEN FELDES, DAMIT DER BENUTZER IM SCHLAFZUSTAND WIE AUF DEM NORD- ODER SÜDPOL LIEGT
DEVICE FOR GENERATING A MAGNETIC FIELD, FIRST VARIABLE AT A FREQUENCY EQUIVALENT TO THE DELTA WAVE, AND THEN CONSTANT, SO THAT THE USER THEN SLEEPS AS IF LOCATED AT THE NORTH OR SOUTH MAGNETIC POLE

(30) Priorité: 16.07.2009 FR 0903486
(43) Date de publication de la demande: 23.05.2012
(73) Titulaire: Beaucamp, Philippe, 77680 Roissy en Brie (FR)
(72) Inventeur: Beaucamp, Philippe, 77680 Roissy en Brie (FR)
(86) Numéro de dépôt international: PCT/FR2010/000509
(87) Numéro de publication internationale: WO 2011/007060

(56) Documents cités:
- EP-A- 0 239 098
- CH-A5- 677 723
- DE-A1- 4 136 374
- DE-A1- 19 747 608
- FR-A- 2 622 808
- FR-A- 2 888 983

## Description

Dispositif créant un champ magnétique d'abord variable à fréquence équivalente au rythme delta, puis constant afin que dès lors l'utilisateur dorme comme placé sur le pôle magnétique Nord -ou Sud.

La présente invention concerne un dispositif permettant de créer un champ magnétique quasiment indépendant du champ magnétique terrestre, dans un premier temps variable, puis constant ; ceci afin de placer l'utilisateur, au début de son sommeil, sur un pôle nord magnétique variable ayant une fréquence équivalente à celle du rythme delta généré par le cerveau pendant le sommeil en stade III et IV, puis, afin qu'au bout d'un temps déterminé, le champ magnétique devenant automatiquement constant, cet utilisateur dorme comme s'il était placé sur le pôle magnétique Nord -ou son inverse Sud.

La présente invention complète le Brevet d'invention dont le numéro de publication INPI est 2 888 983 (N° d'enregistrement national 05 07652)

Le champ magnétique créé par l'invention est variable dans un premier temps avec une fréquence égale ou inférieure à 2 Hz puis devient après une certaine temporisation pratiquement constant sur la surface du lit (F ≈ 1 gauss pour un matelas de 20cm d'épaisseur) et son inclinaison au centre du lit est de 90° (champ vertical). La personne dort alors comme si elle était placée sur le pôle magnétique Nord (ou son inverse Sud si l'on inverse le sens de branchement de la bobine).

En effet, au pôle magnétique Nord, le champ magnétique a pour valeur 0.63 gauss et son inclinaison est égale à 90°.

Selon une première caractéristique, le dispositif comporte au moins un oscillateur produisant une fréquence équivalente à celle du rythme delta généré par le cerveau dans le cas du Sommeil Lent Profond (stade III et IV), soit égale ou inférieure à 2 Hz. Puis, pour passer en champ magnétique constant au bout d'un temps correspondant approximativement à la somme des temps des stades I, II, III et IV, soit environ une heure ; le dispositif comporte également un temporisateur.

Cet oscillateur ainsi que son temporisateur sont placés à l'intérieur du boîtier contentant également la régulation électronique ainsi que l'affichage. Ce boîtier génère le courant circulant dans la bobine créant ce champ magnétique.

Ayant un noyau à air de forme parallélépipédique et une surface identique ou quasi identique à celle du matelas, la bobine est placée à ou sous la périphérie inférieure du matelas, ou sur le pourtour intérieur du sommier, ou sous le socle porte matelas. Elle peut être notamment fixée sur le pourtour d'une membrane.

Le boîtier alimenté par le secteur 50 Hz ou 60 Hz selon les pays, comporte :
- un filtre secteur permettant de protéger l'équipement contre toutes interférences du secteur et également de réduire les interférences de l'équipement susceptibles d'être renvoyées vers le secteur ;
- un circuit d'alimentation basse tension composé au moins d'un transformateur, d'un pont redresseur et d'un condensateur de filtrage ;
- un circuit régulateur de puissance qui permet d'adapter le champ magnétique selon le choix de l'utilisateur et de protéger le système en cas de surintensité ;
- un circuit permettant de régler l'intensité du champ magnétique créé;
- un circuit "Sécurité contre les courts-circuits" détectant les surintensités ;
- un ou plusieurs oscillateurs basses fréquences, ce qui permet de créer un champ magnétique variable, les oscillations étant envoyées dans le circuit "Sommation des paramètres" ; un de ces oscillateurs génère une fréquence égale ou inférieure à 2 Hz, équivalente à la fréquence du rythme delta, ce qui crée donc un champ variable à cette fréquence ;
- un temporisateur calibré sur une durée de l'ordre de 1 heure, qui permet de passer automatiquement du mode champ magnétique variable au mode champ magnétique constant après l'écoulement de ce temps ;
- un circuit qui effectue la sommation des différents paramètres, comme l'intensité du champ magnétique réglé par l'utilisateur, les signaux provenant du ou des oscillateurs, les commandes du temporisateur, la protection contre les courts-circuits, et qui envoie au circuit "régulateur de puissance" et au circuit "affichage" les informations correspondantes ;
- l'affichage, qui peut indiquer le type de champ magnétique créé, le courant circulant dans la bobine et la mise en sécurité de l'appareil.

Selon des modes particuliers de réalisation :
- des fréquences différentes de 2 Hz ou de moins de 2 Hz et pouvant par exemple aller jusqu'à 300 Hz, peuvent être sélectionnées ;
- la durée de la temporisation peut éventuellement être variable ;
- Le temporisateur peut éventuellement être arrêté;
- le filtre secteur ainsi que la partie du circuit d'alimentation basse tension relié au secteur et contenant au minimum le transformateur, peuvent être séparés de ce boîtier et placés dans un autre.

Les dessins annexés illustrent l'invention :
- la figure 1 représente en coupe le dispositif de l'invention, à savoir la bobine (1) placée à la périphérie inférieure du matelas (6), le boîtier (2) comportant le temporisateur, la régulation électronique, l'affichage ainsi que l'oscillateur dont la fréquence est équivalente au rythme delta, soit ≤ 2 Hz ;
- la figure 2 représente le schéma synoptique de l'invention placée dans le boîtier (2) ainsi que la bobine (1).

En référence à ces dessins, le dispositif comporte la bobine (1), placée à la périphérie inférieure du matelas (6), ayant la forme du matelas et une surface identique ou quasi identique à celui-ci. Le boîtier (2) est relié à la bobine par 2 fils de cuivre (3) et alimente celle-ci en basse tension.

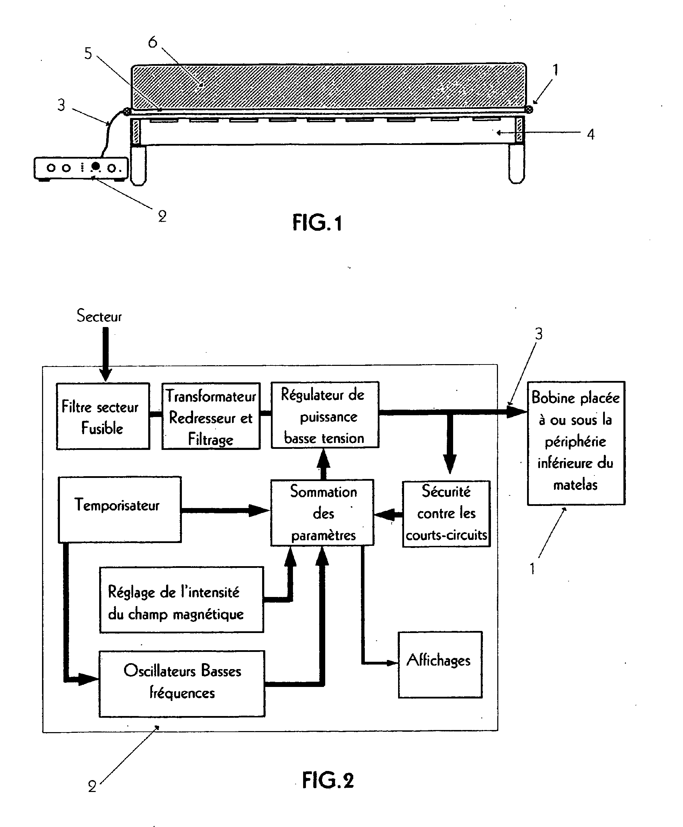

Le schéma synoptique de la figure 2 indique que la bobine (1) est alimentée en basse tension par l'intermédiaire du régulateur de puissance. Celui-ci peut contrôler un courant pouvant atteindre 6A. Un ou plusieurs oscillateurs basses fréquences sont situés dans le boîtier (2) de l'appareil. Un de ces oscillateurs génère une fréquence ≤2 Hz, équivalente au rythme delta. Un temporisateur est calibré pour que l'oscillateur s'arrête et que le champ magnétique devienne constant au bout d'un temps de l'ordre d'une heure. La durée de la temporisation peut éventuellement être variable. Les oscillations, envoyées au circuit de "sommation des paramètres", parviennent donc au régulateur de puissance. Le boîtier (2) peut consommer jusqu'à 120W. Le réglage de l'intensité du champ magnétique peut être effectué par un potentiomètre, ou par une commande numérique, ou par une variation de la tension secondaire du transformateur, ou par tout autre moyen permettant de diminuer l'intensité du courant circulant dans la bobine. La "Sécurité contre les courts-circuits" doit annuler ou limiter fortement l'intensité du courant circulant dans la bobine (1).

## Revendications

1. Dispositif permettant de produire un champ magnétique variable ou constant quasiment indépendant du champ magnétique terrestre, ceci afin qu'allongé sur le matelas (6), l'utilisateur au début de son sommeil soit placé sur un pôle nord variable ayant une fréquence équivalente au rythme delta, soit égale ou inférieure à 2 Hz, puis, afin qu'au bout d'une durée de l'ordre d'une heure, le champ magnétique devenant automatiquement constant, cet utilisateur dorme comme s'il était placé sur le pôle magnétique Nord -ou son inverse Sud- ; le dispositif comportant une bobine (1) placée à ou sous la périphérie inférieure du matelas (6) qui a la forme du matelas et une surface identique ou quasi identique à celui-ci ; la bobine (1) étant alimentée par un boîtier (2) ayant pour fonctions de générer la fréquence égale ou inférieure à 2 Hz, d'effectuer après un temps de l'ordre d'une heure, déterminé grâce à un temporisateur, le basculement entre ledit champ magnétique variable et ledit champ constant, de réguler électroniquement le champ magnétique selon le choix de l'utilisateur, et d'afficher ce choix.

2. Dispositif selon la revendication 1 **caractérisé en ce que** la fréquence de ou des oscillateurs placés dans le boîtier (2) peut être réglée de moins de 2Hz à 300 Hz par l'utilisateur.

3. Dispositif selon l'une quelconque des revendications précédentes **caractérisé en ce que** la durée de la temporisation permettant de passer du champ magnétique variable au champ magnétique constant peut être réglée par l'utilisateur.

4. Dispositif selon l'une quelconque des revendications précédentes **caractérisé en ce que** la bobine (1) est fixée sur le pourtour extérieur d'une membrane (5).

## Claims

1. A device that allows to produce a magnetic field variable or constant, almost independent of earth's magnetic field, in order that stretched out on the mattress (6), the user at the beginning of his sleep is placed on a variable north pole with equivalent frequency of the delta rhythm brain, equal to or less than 2 Hz, and so that after a period of about an hour, the magnetic field automatically becomes constant, so that the user then sleeps as if located at the North Magnetic Pole - or its inverse South - ; the device comprising a coil (1) placed at or below the lower periphery of the mattress (6), with the shape of the mattress and a surface identical or almost identical to this one ; the coil (1) being supplied by a housing (2) whose function is to generate a frequency equal to or less than 2 Hz, to switch after a time of about an hour, determined by a timer, from the aforementioned variable magnetic field to the aforementioned constant field, to regulate electronically the magnetic field according to the choice of the user, and to display that choice.

2. Device according to claim 1, wherein the frequency of the oscillators placed in the housing (2) can be adjusted from less than 2 Hz to 300 Hz by the user.

3. Device according to any preceding claim, wherein the duration of the delay to switch from variable magnetic field to constant magnetic field can be set by the user.

4. Device according to any preceding claim, wherein the coil (1) is fixed to the outer periphery of a membrane (5).

## Patentansprüche

1. Vorrichtung zum Erzeugen eines veränderlichen oder konstanten magnetischen Feldes, das nahezu unabhängig vom Erdmagnetfeld ist. Damit der Benutzer, der auf der Matratze liegt (6), zu Beginn seines Schlafes auf einem veränderlichen Nordpol liegt, mit einer Frequenz äquivalent zum Deltarhythmus, das heißt gleich oder kleiner als 2 Hertz. Damit dann nach etwa einer Stunde das Magnetfeld automatisch konstant wird. Dieser Benutzer schläft, als wäre er auf dem magnetischen Nord- oder Südpol platziert. Die Vorrichtung hat eine Spule (1), die an oder unter der unteren Begrenzung der Matratze (6) angebracht ist, die Form der Matratze hat und eine identische oder fast identische Oberfläche. Die Spule (1), die durch ein Gehäuse (2) versorgt wird, hat die Aufgaben die Frequenz gleich oder niedriger als 2 Hz zu erzeugen. Nach einer gewissen Zeit in der Größenordnung einer Stunde mit Hilfe eines Timers die Umschaltung zwischen dem besagten variablen Magnetfeld und dem konstanten Feld durchzuführen, das Magnetfeld elektronisch entsprechend der Wahl des Benutzers zu regulieren und diese Wahl an zu zeigen.

2. Vorrichtung entsprechend der Anforderung 1, dadurch charakterisiert, dass die Frequenz des oder der Oszillatoren, die die in dem Gehäuse (2) angeordnet sind, durch den Benutzer von weniger als 2 Hz bis 300 Hz eingestellt werden kann.

3. Vorrichtung, nach irgendeiner der vorangehenden Anforderungen, die dadurch charakterisiert ist, dass die Dauer der Verzögerung es erlaubt, durch den Benutzer geregelt zu werden, vom variablen Magnetfeld zum konstanten Magnetfeld überzugehen.

4. Vorrichtung nach irgendeiner der vorangehenden Anforderungen, die dadurch charakterisiert wird, dass die Spule (1) auf dem äußeren Umfang einer Membrane (5) fixiert ist.
